# EUROPEAN PATENT SPECIFICATION

(11) **EP 3 045 451 B1**
(45) Date of publication and mention of the grant of the patent: **28.03.2018**
(21) Application number: 14844485.4
(22) Date of filing: 12.09.2014
(51) Int. Cl.: C07D 277/82, A61K 31/428, A61P 43/00, C12N 5/0735, C12N 5/074

(54) **COMPOUND PROMOTING DIFFERENTIATION OF PLURIPOTENT STEM CELLS INTO CARDIOMYOCYTES**
VERFAHREN ZUR INDUZIERUNG DER DIFFERENZIERUNG PLURIPOTENTER STAMMZELLEN IN KARDIOMYOZYTEN
COMPOSÉ FAVORISANT LA DIFFÉRENCIATION DE CELLULES SOUCHES PLURIPOTENTES EN CARDIOMYOCYTES

(30) Priority: 13.09.2013 JP 2013190462
(43) Date of publication of application: 20.07.2016
(73) Proprietor: Kyoto University, Kyoto-shi, Kyoto 606-8501 (JP)
(72) Inventor: NAKATSUJI, Norio, Kyoto-shi Kyoto 606-8501 (JP); MINAMI, Itsunari, Kyoto-shi Kyoto 606-8501 (JP); UESUGI, Motonari, Kyoto-shi Kyoto 606-8501 (JP); OTSUKA, Shinya, Kyoto-shi Kyoto 606-8501 (JP)
(74) Representative: dompatent von Kreisler Selting Werner - Partnerschaft von Patent- und Rechtsanwälten mbB
(86) International application number: PCT/JP2014/074233
(87) International publication number: WO 2015/037706

(56) References cited:
- WO-A1-2011/002950
- WO-A1-2013/111875
- WO-A1-2014/136519
- JP-A- H0 217 181
- JP-A- S63 190 880
- US-A1- 2014 127 807
- MINAMI, I. ET AL.: 'A small molecule that promotes cardiac differentiation of human pluripotent stem cells under defined, cytokine- and xeno-free conditions' CELL REPORTS vol. 2, no. ISSUE, 2012, pages 1448 - 1460, XP055097336

## Description

### TECHNICAL FIELD

The invention relates to a compound for promoting cardiac differentiation of a pluripotent stem cell and use thereof.

### BACKGROUND

Cardiovascular diseases are the leading cause of death in the world. Cardiac transplantation, which is currently the sole therapeutic option for severe heart failure patients, suffers from donor shortage. A potential therapeutic option as an alternative to cardiac transplantation is transplantation of cardiomyocytes derived from pluripotent stem cells such as iPS and ES cells. The alternative option has been desired to be developed promptly.

Improvement of efficiency and safety is essential to apply iPS cell-derived cardiomyocytes to tissue engineering. Regarding efficiency, current methods cannot induce a large number of cardiomyocytes and they are not cost-effective because proteins added to medium such as growth factors are very expensive. Regarding safety, cancer development is concerned because current methods are difficult to provide cardiomyocytes of high purity and thus proliferating cells other than cardiomyocytes can be contaminated.

To overcome such problems, the inventors of the present application have developed the compounds that have an activity to promote cardiac differentiation of pluripotent stem cells (Patent Literatures 1 and Non Patent Literature 1, the references are herein incorporated by reference). In particular, a novel compound KY02111 is a potent promoter compared to the compounds and proteins that have been known to promote cardiac differentiation.

KY02111 is quite useful as a promoter of cardiac differentiation of pluripotent stem cells. However, to obtain sufficient cardiac differentiation, KY02111 is added to medium at a relatively high concentration. For further reducing costs and improving safety, compounds that promote cardiac differentiation at a lower concentration would be desirable.

### CITATION LIST

### PATENT LITERATURE

Patent Literature 1: WO2012/026491
Patent Literature 2: WO2013/111875

### NON PATENT LITERATURE

Non Patent Literature 1: Minami, I, et al., Cell Reports, Volume 2, Issue 5, 1448-1460, 2012
Non Patent Literature 2: Graichen, R., et al., Differentiation 76, 357-370, 2008
Non Patent Literature 3: Hao, J., et al., PLoS One 3, e2904, 2008
Non Patent Literature 4: Wang, H., Hao, J., and Hong, C.C., ACS Chem. Biol. 6, 192-197, 2011
Non Patent Literature 5: Ren, Y., et al., J. Mol. Cell. Cardiol. 51, 280-287, 2011
Non Patent Literature 6: Willems, E., et al., Circ. Res. 109, 360-364, 2011
Non Patent Literature 7: Lian, X., et al., Proc. Natl. Acad. Sci. USA 109, E1848-E1857, 2012

The above references are herein incorporated by reference.

### SUMMARY

An object of the invention is to provide a compound that enables more efficient and less costly production of cardiomyocytes from pluripotent stem cells than KY02111.

The inventors have synthesized compounds that have a different halogen group from Cl or a different carbon chain length (n) compared to KY02111, and compared the effect of promoting cardiac differentiation between the synthesized compounds and KY02111. The inventors have identified some compounds that promote cardiac differentiation at a much lower concentration than KY02111. The invention is thus achieved.

The invention provides:
1. A compound having Formula (II) 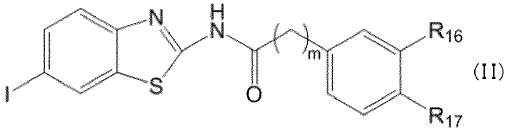 wherein
   m is 1 to 4, and
   R₁₆ and R₁₇ are independently selected from methoxy, ethoxy and propoxy,
   or a salt thereof.
2. The compound of item 1 or a salt thereof, wherein R₁₆ is methoxy.
3. The compound of item 2 or a salt thereof, wherein R₁₇ is methoxy or propoxy.
4. The compound of any one of items 1-3 or a salt thereof, wherein m is 1 to 3.
5. A compound having any one of the following formulas: 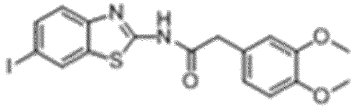 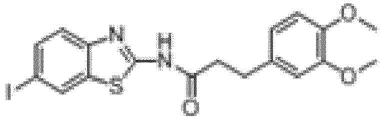 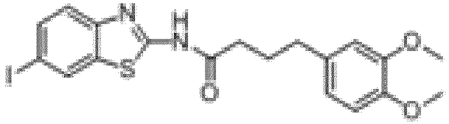 and 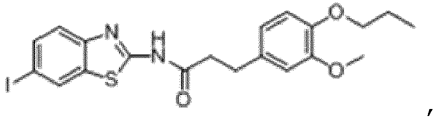 or a salt thereof.
6. A composition for promoting cardiac differentiation of a pluripotent stem cell comprising the compound of any one of items 1-5 or a salt thereof.
7. The composition for promoting cardiac differentiation of a pluripotent stem cell of item 6, wherein the composition comprises the compound 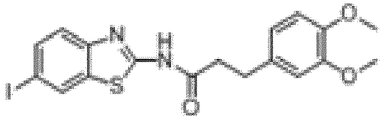 or 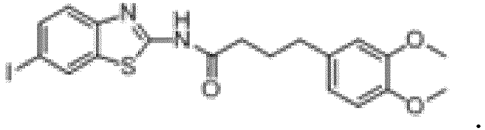
8. The composition for promoting cardiac differentiation of a pluripotent stem cell of item 6 or 7, wherein the composition is added to cardiac differentiation medium to provide a final concentration of the compound of 0.4 to 2 µM.
9. A kit for promoting cardiac differentiation of a pluripotent stem cell comprising the compound of any one of items 1-5 or a salt thereof.
10. A method for inducing cardiac differentiation of a pluripotent stem cell, comprising culturing the pluripotent stem cell in a medium comprising the compound of any one of items 1-5 or a salt thereof.
11. The method of item 10, wherein the method comprises culturing the pluripotent stem cell in a medium comprising the compound 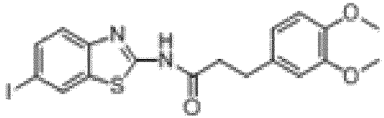 or 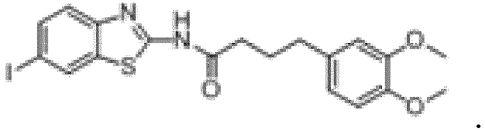
12. The method of item 10 or 11, wherein the medium comprises the compound at 0.4 to 2 µM.
13. A method for preparing a cardiomyocyte from a pluripotent stem cell comprising culturing the pluripotent stem cell in a medium comprising the compound of any one of items 1-5 or a salt thereof.
14. The method of item 13, wherein the method comprises culturing the pluripotent stem cell in a medium comprising the compound 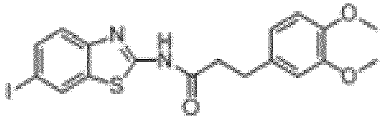 or 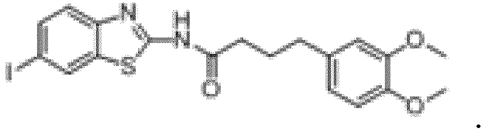
15. The method of item 13 or 14, wherein the medium comprises the compound at 0.4 to 2 µM.

The invention enables more efficient and less costly production of cardiomyocytes from pluripotent stem cells.

### BRIEF DESCRIPTION OF THE DRAWINGS

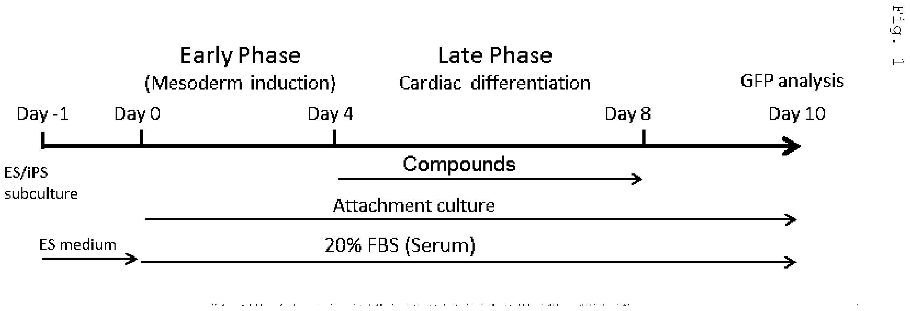

Fig. 1 shows protocol for analyzing the effect of promoting cardiac differentiation in monkey ES cells.
Fig. 2 shows structure-activity relationship of R₇ substitution analogues in the effect of promoting cardiac differentiation.
Fig. 3 shows structure-activity relationship about the carbon chain length (n) of iodine substitution analogues.
Fig. 4 shows comparison between iodine substitution analogues having the carbon chain lengths of 2 and 3.
Fig. 5 shows structure-activity relationship about R₂ and R₃ groups of iodine substitution analogues.
Fig. 6 shows the effect of the iodine substitution analogue SO2031 (KY02-I) for promoting cardiac differentiation in human ES/iPS cells.
Fig. 7 shows the solubility of the iodine substitution analogue SO2031 (KY02-I) in medium. A: serum-containing medium (20 µM), B: serum-free medium (20 µM), C: serum-free medium (3 µM).

### DESCRIPTION OF EMBODIMENTS

The compound disclosed in WO2012/026491 (the reference is herein incorporated by reference.) has Formula (I): 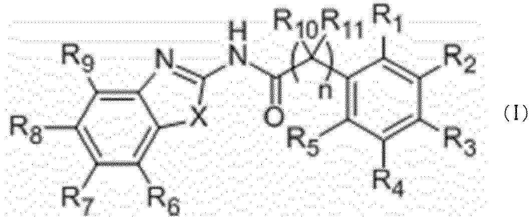 wherein
R₁ to R₅ are each independently hydrogen; halogen; hydroxyl; linear or branched alkoxy having 1 to 5 carbon atoms; linear or branched alkyl having 1 to 5 carbon atoms which is unsubstituted or substituted with halogen; or - NR₁₂R₁₃, wherein R₁₂ and R₁₃ are each independently hydrogen, oxygen, or linear or branched alkyl having 1 to 5 carbon atoms which is unsubstituted or substituted with halogen; wherein two adjacent groups among R₁ to R₅ may join together to form -O-CH₂-O- or -O-(CH₂)₂-O-,
R₆ to R₉ are each independently hydrogen; halogen; hydroxyl; linear or branched alkoxy having 1 to 5 carbon atoms; linear or branched alkoxy having 1 to 5 carbon atoms which is substituted with -C(O)A, wherein A is a saturated or unsaturated 5- or 6-membered ring which is unsubstituted or substituted with linear or branched alkyl having 1 to 5 carbon atoms and the ring may contain 1 or 2 atoms independently selected from nitrogen, oxygen and sulfur; linear or branched alkyl having 1 to 5 carbon atoms which is unsubstituted or substituted with halogen; or -NR₁₂R₁₃ wherein R₁₂ and R₁₃ are each independently hydrogen, oxygen, or linear or branched alkyl having 1 to 5 carbon atoms which is unsubstituted or substituted with halogen; wherein two adjacent groups among R₆ to R₉ may join together to form -O-CH₂-O- or -O-(CH₂)₂-O-,
R₁₀ to R₁₁ are each independently hydrogen; or linear or branched alkyl having 1 to 5 carbon atoms,
X is -CR₁₄, wherein R₁₄ is hydrogen, halogen, hydroxyl, linear or branched alkoxy having 1 to 5 carbon atoms, or linear or branched alkyl having 1 to 5 carbon atoms which is unsubstituted or substituted with halogen; oxygen; sulfur; selenium; or -NR₁₅, wherein R₁₅ is hydrogen, linear or branched alkyl having 1 to 5 carbon atoms, or linear or branched acyl having 1 to 5 carbon atoms, and
n is an integer of 0 to 6.

KY02111 is a compound having the following formula:

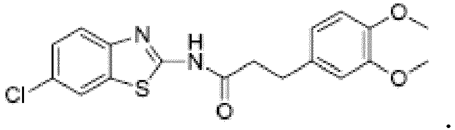

In contrast, the compound of the invention has Formula (II) 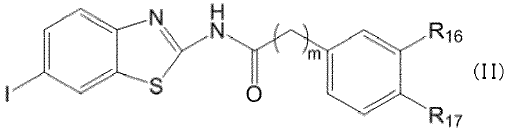 wherein
m is 1 to 4, and
R₁₆ and R₁₇ are independently selected from methoxy, ethoxy and propoxy.

In a preferred embodiment, R₁₇ is methoxy, ethoxy or propoxy and R₁₆ is methoxy. In a more preferred embodiment, R₁₇ is methoxy or propoxy and R₁₆ is methoxy.

In a preferred embodiment, m is 1 to 3, more preferably 2 or 3.

In a preferred embodiment, the compound of the invention is selected from 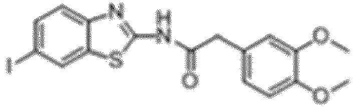 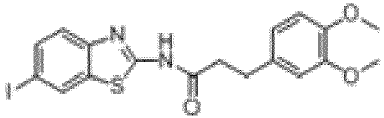 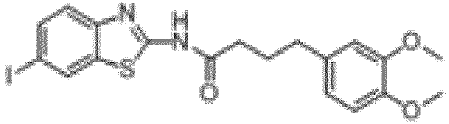 and

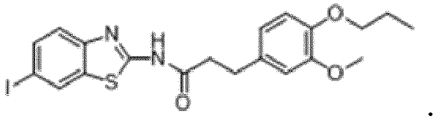

Examples of the salt of the compound of the invention include sodium salt, potassium salt, calcium salt, magnesium salt, and ammonium salt.

The compound of the invention may be synthesized in accordance with the methods described in Examples.

The term "pluripotent stem cell" herein used refers to a cell having an ability to differentiate any type of cell constituting an adult body (pluripotency) and self-renewal capacity which is an ability to maintain the pluripotency during cell division. The "pluripotent stem cell" includes an embryonic stem cell (ES cell), an embryonic germ cell (EG cell), and an induced pluripotent stem cell (iPS cell). The "pluripotent stem cell" may be a cell of any species with no limitation, and preferably a mammalian cell, and more preferably a rodent or primate cell. The present invention is particularly suitable for a monkey or human pluripotent stem cell, in particular a monkey or human ES or iPS cell.

An ES cell is a pluripotent stem cell derived from early embryo and may be established from inner cell mass of a blastocyst or post-implantation epiblast in early embryo. Examples of the ES cell include those described in the following references: human (Thomson J. A. et al., Science 282: 1145-1147 (1998); Biochem Biophys Res Commun. 345(3), 926-32 (2006); primates such as rhesus macaque and marmoset (Thomson J. A. et al., Proc. Natl. Acad. Sci. USA 92: 7844-7848 (1995); Thomson J. A. et al., Biol. Reprod. 55: 254-259 (1996)); rabbit (JP2000-508919A); hamster (Doetshman T. et al., Dev. Biol. 127: 224-227 (1988)), hog (Evans M. J. et al., Theriogenology 33: 125128 (1990); Piedrahita J. A. et al., Theriogenology 34: 879-891 (1990); Notarianni E. et al., J. Reprod. Fert. 40: 51-56 (1990); Talbot N. C. et al., Cell. Dev. Biol. 29A: 546-554 (1993)), sheep (Notarianni E. et al., J. Reprod. Fert. Suppl. 43: 255-260 (1991)), cow (Evans M. J. et al., Theriogenology 33: 125-128 (1990); Saito S. et al., Roux. Arch. Dev. Biol. 201: 134-141 (1992)), and mink (Sukoyan M. A. et al., Mol. Reorod. Dev. 33: 418-431 (1993)) (these references are herein incorporated by reference).

An EG cell is a pluripotent stem cell derived from a primordial germ cell, and examples of the EG cell include a human EG cell (Shamblott, et al., Proc. Natl. Acad. Sci USA 95: 13726-13731 (1998)) (the reference is herein incorporated by reference.).

The term "iPS cell" herein used refers to a pluripotent stem cell induced from a cell other than a pluripotent stem cell such as a somatic cell and a tissue stem cell. Methods for preparing the iPS cell are described in the following references, for example: WO2007/069666, WO2009/006930, WO2009/006997, WO2009/007852, WO2008/118820, Cell Stem Cell 3(5): 568-574 (2008), Cell Stem Cell 4(5): 381-384 (2009), Nature 454: 646-650 (2008), Cell 136(3): 411-419 (2009), Nature Biotechnology 26: 1269-1275 (2008), Cell Stem Cell 3: 475-479 (2008), Nature Cell Biology 11: 197-203 (2009), Cell 133 (2) : 250-264 (2008), Cell 131(5): 861-72 (2007), Science 318 (5858): 1917-20 (2007) (these references are herein incorporated by reference.). However, the "iPS cell" of the present invention includes any cell prepared by any method as long as the cell is a pluripotent stem cell that has been induced artificially.

The compound of the invention is added to a cardiac differentiation medium at a final concentration of 0.1 to 20 µM, preferably 0.1 to 10 µM, more preferably 0.4 to 2 µM, still more preferably 0.4 to 1 µM. The cardiac differentiation medium may be any conventional medium used for cardiac differentiation of pluripotent stem cells and the composition of the medium is not limited to specific compositions. Examples of the medium include IMDM-based cardiac differentiation medium (for example, the medium used in Examples), DMEM-based cardiac differentiation medium (for example, a medium containing 200 ml of DMEM/F12 medium (Sigma), 50 ml of bovine fetal serum (GIBCO), 2.5 ml of MEM non-essential amino acid solution (Sigma), 2.5 ml of penicillin-streptomycin (GIBCO), 2.5 ml of 200 mM L-glutamine, and 2-mercaptoethanol), and StemPro-34SFM (GIBCO) + BMP4 (10 ng/ml).

The compound of the invention may be used in any conventional culture method suitable for cardiac differentiation of a pluripotent stem cell. Examples of the culture method include adhesion culture, floating culture, and suspension culture.

The period from the start of culture in a cardiac differentiation medium (i.e., culture for cardiac differentiation) to the start of culture in a medium containing the compound of the invention, and the period of the culture in a medium containing the compound of the invention may be appropriately determined depending on the types of pluripotent stem cells and the composition of cardiac differentiation medium to be used. For example, when the cell is a primate pluripotent stem cell, in particular when a monkey or human ES or iPS cell is cultured in the IMDM-based cardiac differentiation medium used in Examples, the cell may be cultured in a medium containing the compound of the invention for two days or more within the period from Day 2 to Day 14 of culture for cardiac differentiation (specifically, for 2, 3, 4, 5, 6, 7, 8, 9, 10, 11, or 12 days), preferably for 3 to 10 days, more preferably for 4 to 10 days, still more preferably for 4 to 8 days, still more preferably for 4 to 6 days. In particular, the cell is preferably cultured in a medium containing the compound of the invention for 4 to 6 days up until Day 10 of culture for cardiac differentiation, for example from Day 3 to Day 9, Day 3 to Day 8, Day 3 to Day 7, Day 4 to Day 10, Day 4 to Day 9 or Day 4 to Day 8 of culture for cardiac differentiation.

The compound of the invention may be used in combination with a different cardiac differentiation promoter(s) such as nitrovin, cytokines (combination of bFGF, BMP4, VEGF, DKK1 and Activin A), or Wnt signaling inhibitors. The "WNT signaling inhibitor" refers to a substance which inhibits the WNT signaling pathway. Examples of the WNT signaling inhibitor include compounds such as IWP2, XAV939, and IWR1, and proteins such as IGFBP4 and Dkk1. The term "cardiac differentiation promoter" as used herein refers to any substance which has an effect to promote cardiac differentiation.

The compound of the invention may be used according to a method comprising (1) culturing a pluripotent stem cell in a medium containing one or more WNT signaling activators, and (2) culturing the cell obtained after the step (1) in a medium containing the compound of the invention.

The "WNT signaling activator" refers to a substance which activates the WNT signaling pathway. Examples of the WNT signaling activator include a GSK3β inhibitor such as BIO or CHIR99021. In the method as described above, two or more, for example 2, 3, or 4 WNT signaling activators may be used in combination.

The above method enables efficient induction of cardiac differentiation of a pluripotent stem cell even when the medium does not contain serum (i.e., when "a serum-free medium" is used). When a serum-free medium is used, the medium preferably contains albumin and the cell is preferably cultured by adhesion culture, although it may be cultured by any culture method such as adhesion culture, floating culture, and suspension culture. Examples of albumin include bovine serum albumin and human serum albumin. In the adhesion culture, the culture dish may be coated with gelatin or laminin (such as human laminin 211). The serum-free medium containing albumin enables induction of cardiac differentiation of a pluripotent stem cell in the absence of proteins other than albumin and components derived from species of organism different from the pluripotent stem cell (i.e., xenogeneic components), such as serum, cytokines, and feeder cells.

In the above method, the period from the start of culture for cardiac differentiation to the start of the step (1) or (2) and the period of each of the steps (1) and (2) may be appropriately determined. The step (2) may be started just after the end of the step (1), or after a certain period from the end of the step (1). The WNT signaling activator and the compound of the invention may be added at early and middle phases of cardiac differentiation of a pluripotent stem cell, respectively. The early phase of cardiac differentiation of a pluripotent stem cell is a stage at which differentiation of a pluripotent stem cell into mesoderm is induced and the expression of a mesoderm marker gene is increased. The middle phase of cardiac differentiation of a pluripotent stem cell is a stage at which differentiation of mesoderm into cardiac muscle is induced. Examples of the mesoderm marker includes T, MIXL1, and NODAL. For example, when the cell is a primate pluripotent stem cell, in particular monkey or human ES or iPS cell, the step (1) may be conducted at Day 0 to Day 2 or Day 0 to Day 3 of culture for cardiac differentiation, in other words, for 2 or 3 days from the start of culture for cardiac differentiation, and the step (2) may be conducted, up until Day 14 of culture for cardiac differentiation, for 2 days or more (specifically, for 2, 3, 4, 5, 6, 7, 8, 9, 10, 11, or 12 days), preferably for 3 to 10 days, more preferably for 4 to 10 days, still more preferably for 4 to 8 days, still more preferably for 4 to 6 days. Preferably, the step (2) is conducted for 4 to 6 days up until Day 10 of culture for cardiac differentiation, for example Day 3 to Day 9, Day 3 to Day 8, Day 3 to Day 7, Day 4 to Day 10, Day 4 to Day 9, or Day 4 to Day 8 of culture for cardiac differentiation.

The WNT signaling activator may be used at an appropriate concentration, not at particularly specific concentrations. When the WNT signaling activator is BIO or CHIR99021, the WNT signaling activator may be used at a final concentration of 100 nM to 100 µM, preferably 1 µM to 10 µM. The compound of the invention may be used at a final concentration of 0.1 to 20 µM, preferably 0.1 to 10 µM, more preferably 0.4 to 2 µM, still more preferably 0.4 to 1 µM.

The compound of the invention may be used to prepare a cardiomyocyte. Differentiation into a cardiomyocyte may be detected from the number of beating cardiac colonies, expression of a cardiac marker, expression of an ion channel, or a response to an electrophysiological stimulus. Examples of the cardiac marker include α-MHC, β-MHC, cTnT, α-actinin, and NKX2.5. Examples of the ion channel include HCN4, Nav1.5, Cav1.2, Cav3.2 HERG1b and KCNQ1. The cardiomyocyte prepared by the method of the invention may be used to evaluate drug safety *in vitro* or as a cardiomyocyte for transplant to treat heart diseases.

The invention also provides a composition or a kit for promoting cardiac differentiation comprising the compound of the invention or a salt thereof. The kit of the invention may comprise a different cardiac differentiation promoter(s) in addition to the compound of the invention. The compound of the invention and the different cardiac differentiation promoter(s) may be stored in separate containers or in a single container.

The invention further provides a method for inducing cardiac differentiation of a pluripotent stem cell comprising culturing the pluripotent stem cell in a medium comprising the compound of the invention or a salt thereof, and a method for preparing a cardiomyocyte from a pluripotent stem cell comprising culturing the pluripotent stem cell in a medium comprising the compound of the invention or a salt thereof. The methods can be carried out according to the description of how to use of the compound of the invention.

The invention further provides use of the compound of the invention or a salt thereof for preparing a composition or kit for promoting cardiac differentiation of a pluripotent stem cell.

The invention is described further in detail with reference to the following examples.

### EXAMPLES

### 1. Strategy for analyzing the effect of promoting cardiac differentiation in monkey ES cells

Compounds having a different substituent at the position of Cl of KY02111, which corresponded to R₇ of Formula I, such as a halogen atom other than Cl, were synthesized, and examined for the effect of promoting cardiac differentiation in a dose-dependent manner in monkey ES cells as described previously (Cell Reports, Volume 2, Issue 5, 1448-1460, 25 October 2012; and WO2012/026491, which are herein incorporated by reference). Specifically, a vector expressing green fluorescent protein (GFP) under control of promoter of α-MHC gene, a marker of cardiac differentiation, was introduced into monkey ES cells (CMK 6.4 cynomolgus monkey ES cell line) and the cells were seeded on 6-well culture plates (Asahi Glass/5816-006 : Ezview culture plate) at 4.0 x 10⁵ cells/well, and cultured in an IMDM-based cardiac differentiation medium (200 ml IMDM (Sigma) containing 50 ml bovine fetal serum (GIBCO), 2.5 ml MEM non-essential amino acid solution (Sigma), 2.5 ml penicillin-streptomycin (GIBCO), 2.5 ml 200 mM L-glutamine, 2 µl 2-mercaptoethanol). Each compound was added to the cells on days 4-8 of the cardiac differentiation culture, and the GFP fluorescence was analyzed on day 10 by using MetaMorph imaging system.

### 2. Structure-activity relationship of R₇ substitution analogues in the effect of promoting cardiac differentiation

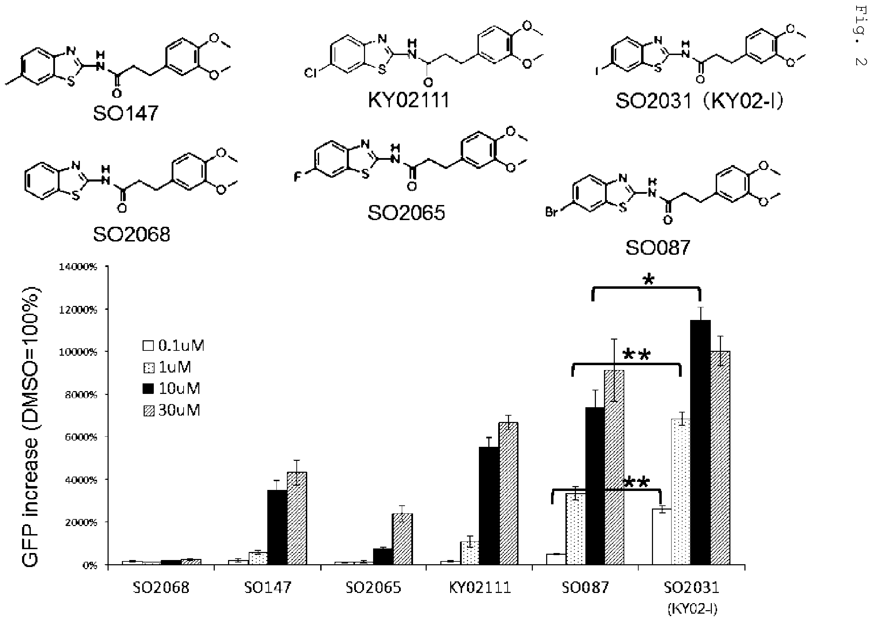

The analysis of item 1 above revealed that the effect of promoting cardiac differentiation increased in the order of the compounds having H, F, CH₃, Cl, Br, and I (H<F<CH₃<Cl<Br<I) at R₇ position (Fig. 2). In particular, SO2031 (referred to as KY02-I hereinafter), which was an iodine substitution analogue, showed the effect about 16, 6.3, 2.1, and 1.5 times higher than that of KY02111 having Cl at R₇ position in 0.1 µM, 1 µM, 10 µM, and 30 µM, respectively. Further, SO2031 (KY02-I) showed the effect about 5.2, 2, 1.6, and 1.1 times higher than that of SO087 having Br at R₇ position in 0.1 µM, 1 µM, 10 µM, and 30 µM, respectively. These results demonstrate that the iodine substitution analogue is the most effective especially in a lower dose.

### 3. Structure-activity relationship about the carbon chain length of iodine substitution analogues

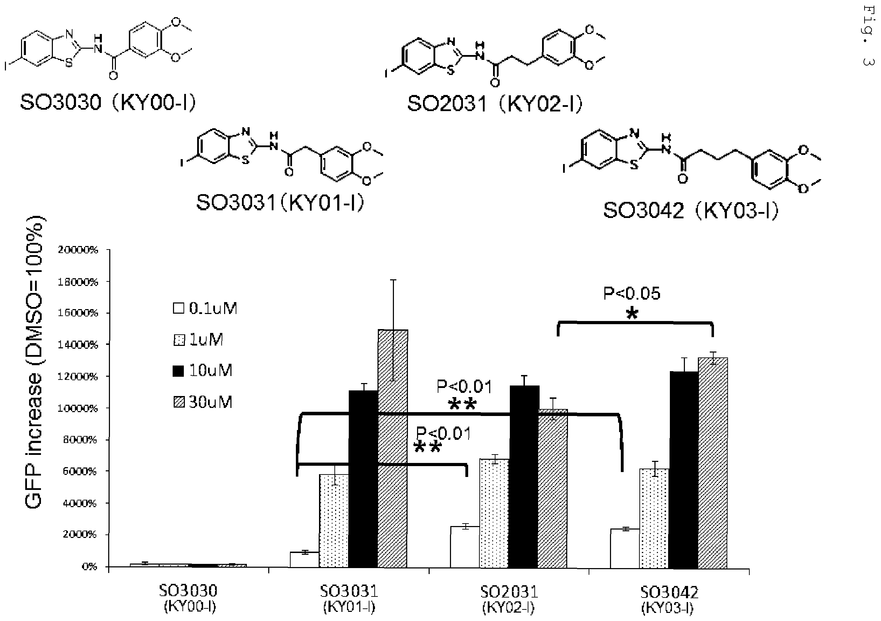

Compounds each having a carbon chain length (n) different from that of KY02-I were synthesized and examined for the effect of promoting cardiac differentiation (Fig. 3). SO3031 (KY01-I) having a carbon chain length of 1, SO2031 (KY02-I) having a carbon chain length of 2, and SO3042 (KY03-I) having a carbon chain length of 3 showed a higher effect than KY02111, while SO3030 (KY00-I) having a carbon chain length of 0 showed almost no effect. In addition, at a low dose (0.1 µM), SO2031 (KY02-I) having a carbon chain length of 2 and SO3042 (KY03-I) having a carbon chain length of 3 showed a higher effect than SO3031 (KY01-I) having a carbon chain length of 1 (2.7 and 2.8 times, respectively). Further, at a high dose (10 µM or 30 µM), SO3042 (KY03-I) showed a slightly higher effect than SO2031 (KY02-I) (1.3 times).

### 4. Comparison between iodine substitution analogues having the carbon chain lengths of 2 and 3

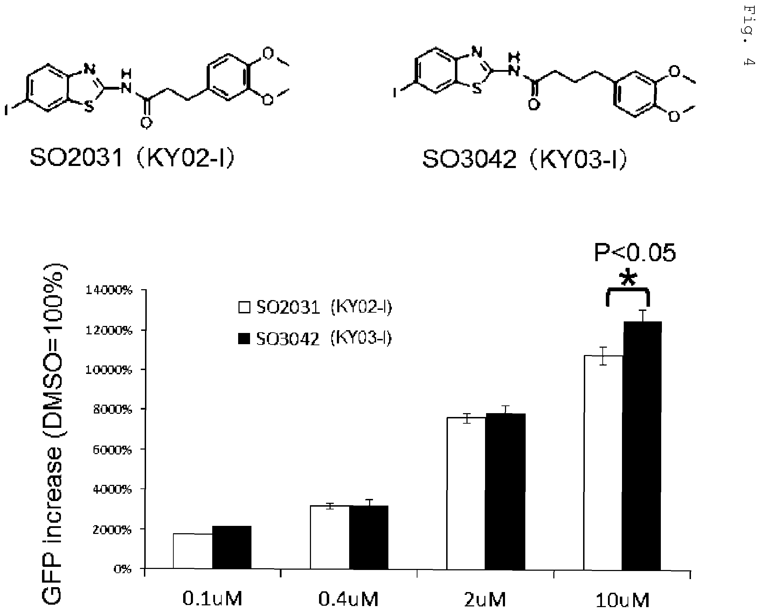

The effect of promoting cardiac differentiation of SO2031 (KY02-I) having a carbon chain length of 2 and SO3042 (KY03-I) having a carbon chain length of 3 was confirmed in a separate experiment from Fig. 3 in a dose-dependent manner (Fig. 4). At 0.1 µM, 0.4 µM and 2 µM, these compounds showed a comparable effect, and at a higher dose (10 µM), SO3042 (KY03-I) showed a slightly higher effect than SO2031 (KY02-I). Together with the result of Fig. 3, SO3042 (KY03-I) having a carbon chain length of 3 has the effect of promoting cardiac differentiation slightly higher than that of SO2031 (KY02-I) at a higher dose.

### 5. Structure-activity relationship about R₂ and R₃ groups of iodine substitution analogues

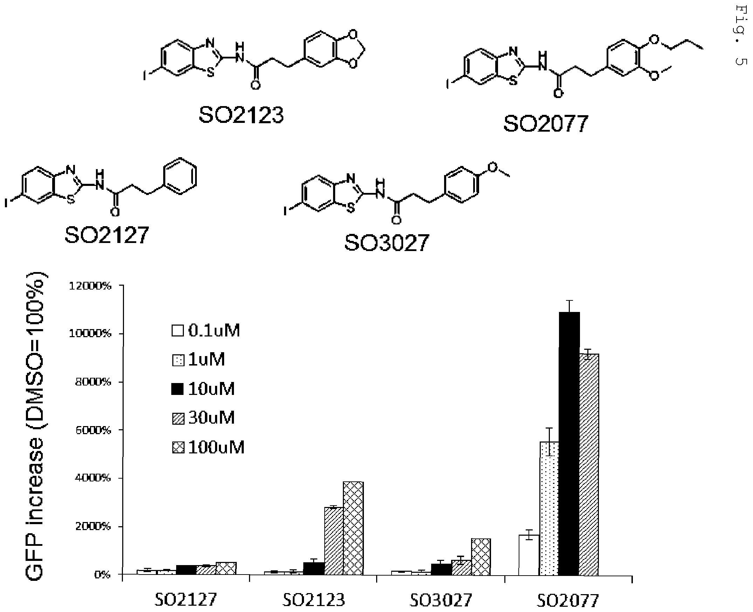

Compounds having different groups at the positions of the methoxy groups of KY02111, which corresponded to R₂ and R₃ of Formula I, were synthesized and examined for the effect of promoting cardiac differentiation (Fig. 5). The effect of promoting cardiac differentiation was decreased in the compounds having no dimethoxy structure except for SO2077 having a propoxy group at the position R₂ instead of a methoxy group. SO2077 showed the effect comparable to that of SO2031 (KY02-I) having the dimethoxy structure.

### 6. Effect of the iodine substitution analogue SO2031 (KY02-I) for promoting cardiac differentiation in human ES/iPS cells

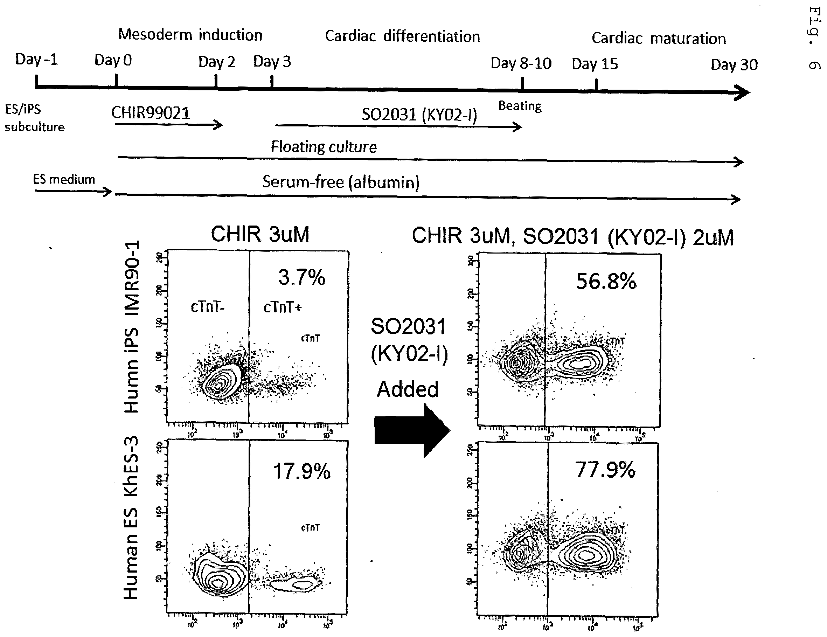

The effect of promoting cardiac differentiation of SO2031 (KY02-I) was confirmed using human ES cells (KhES-3) and iPS cells (IMR90-1) (Fig. 6). Induction of cardiac differentiation was performed as described previously (Cell Reports, Volume 2, Issue 5, 1448-1460, 25 October 2012, which is herein incorporated by reference). Specifically, IMDM (Sigma) containing 1% MEM non-essential amino acid solution (Sigma), 1% penicillin-streptomycin (Gibco), 2 mM L-glutamine (Sigma), 0.5 mM L-carnitine (Sigma), 0.001% 2-mercaptoethanol (Gibco), and 0.4% human serum albumin (Sigma) was used for culture. Cardiac differentiation was induced in Ultra-low attachment culture dish (Corning) by floating culture. During the first two days, 3 µM CHIR99021, a WNT activator, was added, and on days 3-8 of culture, 2 µM SO2031 (KY02-I) was added. Efficiency of cardiac differentiation was determined by calculating the percentage of cardiomyocytes by flow cytometry using an antibody for cardiac troponin T (cTnT), a cardiac-specific marker molecule. SO2031 (KY02-I) increased the ratio of cardiomyocytes from 3.7 % to 56.8% in human iPS cells (IMR90-1), and from 17.9% to 77.9% in human ES cells (KhES-3). These results demonstrate that the iodine substitution analogue SO2031 (KY02-I) efficiently promotes cardiac differentiation at a lower dose without stimulation by cytokines and growth factors not only in monkey ES cells but also in human ES/iPS cells.

### 7. Solubility of the iodine substitution analogue SO2031 (KY02-I) in medium

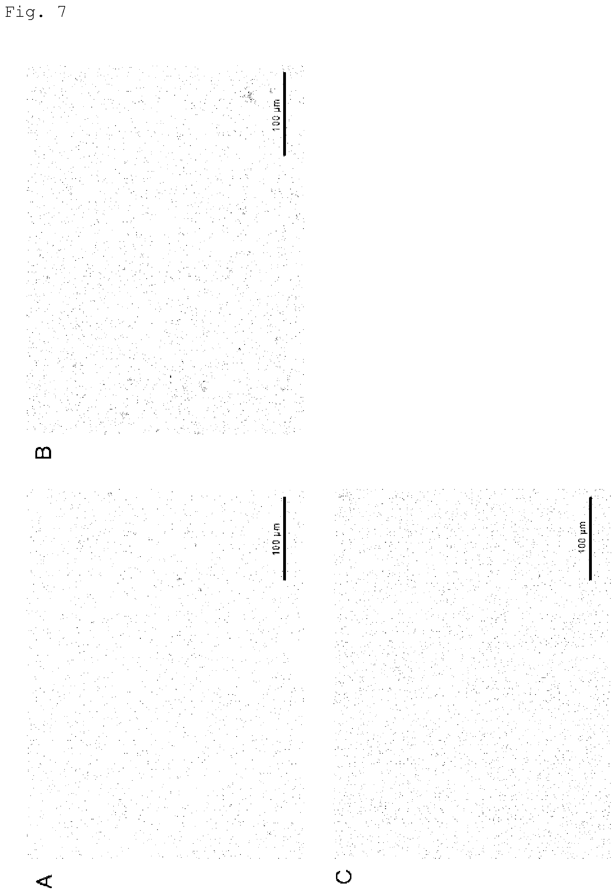

SO2031 (KY02-I) was dissolved in 20% serum-containing medium (200 ml IMDM (Sigma) containing 50 ml bovine fetal serum (GIBCO), 2.5 ml MEM non-essential amino acid solution (Sigma), 2.5 ml penicillin-streptomycin (GIBCO), 2.5 ml 200 mM L-glutamine, 2 µl 2-mercaptoethanol) at 20 µM, or in serum-free medium (200 ml IMDM (Sigma) containing 2.5 ml MEM non-essential amino acid solution (Sigma), 2.5 ml penicillin-streptomycin (GIBCO), 2.5 ml 200 mM L-glutamine, 2 µl 2-mercaptoethanol) at 3 or 20 µM and observed after 24 hours (Fig. 7). Large crystals were not observed in any of the media (Fig. 7A-C). Also, in the serum-free medium, almost no crystal was observed at 3 µM, at which concentration a sufficient promotion of cardiac differentiation was obtained. Since the compound is not crystallized, concentration of the compound in the medium would not be decreased by precipitation of crystals and would be stably maintained. Also, there would be no possibility that precipitated crystals affect the cells. Further, there would be no risk that crystals remaining in the cells are transferred to and affect the host at transplantation.

### 8. Preparation Examples

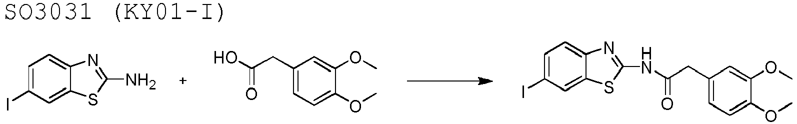

N,N-dimethylformamide solution (3ml) containing 2-amino-6-iodobenzothiazole (200 mg, 0.723 mmol) and 3,4-dimethoxyphenylacetic acid (157 mg, 0.795 mmol) was added with N,N-diisopropylethylamine (139 µl, 0.803 mmol) and O-(6-chlorobenzotriazol-1-yl)-N,N,N',N'-tetramethyluronium hexafluorophosphate (360 mg, 0.870 mmol) and stirred over night at room temperature. After completion of the reaction, the solution was diluted with ethyl acetate and washed with saturated sodium bicarbonate solution and saturated sodium chloride solution. The solution was dried over anhydrous sodium sulfate and the solvent was removed under reduced pressure. The residue was recrystallized with ethanol and 167 mg of 2-(2-(3,4-dimethoxyphenyl)acetamide)-6-iodobenzothiazole was obtained in a yield of 50%.
¹H NMR (DMSO-d₆) : δ 12.61 (s, 1H), 8.37 (s, 1H), 7.73-7.69 (m, 1H), 7.54 (d, J = 8.0 Hz, 1H), 6.97-6.84 (m, 3H), 3.75-3.72 (m, 8H).
MS (ESI) Found; 455[M+H]⁺

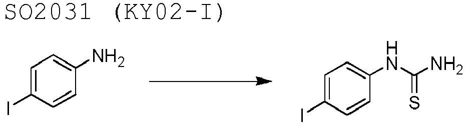

Dichloromethane solution (3 ml) of 4-iodoaniline (1.00 g, 4.57 mmol) was added with thiocarbonyldiimidazole (976 mg, 5.47 mmol) and stirred for 1.5 hours at room temperature. After addition of 25% ammonia solution (3ml), the solution was stirred over night at room temperature. After completion of the reaction, the solvent was removed under reduced pressure, and the resulting deposits were filtered to obtain 889 mg of 1-(4-iodophenyl)thiourea at a yield of 59%.

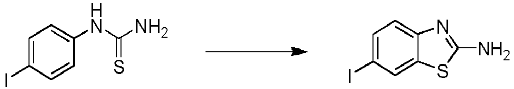

Chloroform suspension (7ml) of 1-(4-iodophenyl)thiourea (889 mg, 3.19 mmol) was added with bromine (328 µl, 6.40 mmol), and heated to reflux and stirred for 6 hours. After the reaction was completed and the solvent was removed, the residue was added with dichloromethane and washed with saturated sodium bicarbonate solution and saturated sodium chloride solution. After the solution was dried over anhydrous sodium sulfate and the solvent was removed under reduced pressure, the resulting deposits were filtered to obtain 650 mg 2-amino-6-iodobenzothiazole in a yield of 73%.

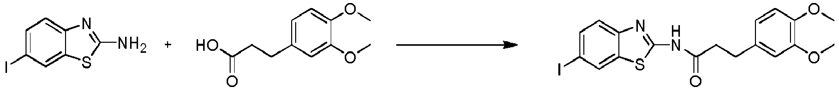

N,N-dimethylformamide solution (2ml) containing 2-amino-6-iodobenzothiazole (100 mg, 0.362 mmol) and 3-(3,4-dimethoxyphenyl)propionic acid (91.4 mg, 0.435 mmol) was added with N,N-diisopropylethylamine (69.4 µl, 0.398 mmol) and O-(6-chlorobenzotriazol-1-yl)-N,N,N',N'-tetramethyluronium hexafluorophosphate (180 mg, 0.435 mmol) and stirred over night at room temperature. After completion of the reaction, the solution was diluted with ethyl acetate and washed with saturated sodium bicarbonate solution and saturated sodium chloride solution. The solution was dried over anhydrous sodium sulfate and the solvent was removed under reduced pressure. The residue was recrystallized with ethanol and 83 mg of 2-(3-(3,4-dimethoxyphenyl)propanamide)-6-iodobenzothiazole in a yield of 48%.
¹H NMR (DMSO-d₆) : δ 12.42 (s, 1H), 8.37 (s, 1H), 7.72-7.69 (m, 1H), 7.52 (d, J = 8.4 Hz, 1H), 6.85-6.83 (m, 2H), 6.75-6.72 (m, 1H), 3.71 (s, 3H), 3.69 (s, 3H), 2.90-2.76 (m, 4H). MS (ESI) Found; 469[M+H]⁺

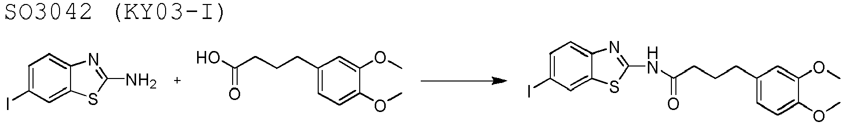

N,N-dimethylformamide solution (3ml) containing 2-amino-6-iodobenzothiazole (250 mg, 0.905 mmol) and 4-(3,4-dimethoxyphenyl)butanoic acid (224 mg, 0.995 mmol) was added with N,N-diisopropylethylamine (174 µl, 0.995 mmol) and O-(6-chlorobenzotriazol-1-yl)-N,N,N',N'-tetramethyluronium hexafluorophosphate (450 mg, 1.09 mmol) and stirred over night at room temperature. After completion of the reaction, the solution was diluted with ethyl acetate and washed with saturated sodium bicarbonate solution and saturated sodium chloride solution. The solution was dried over anhydrous sodium sulfate and the solvent was removed under reduced pressure. The residue was recrystallized with ethanol and 131 mg of 2-(4-(3,4-dimethoxyphenyl)butanamide)-6-iodobenzothiazole was obtained in a yield of 30%.
¹H NMR (DMSO-d₆) : δ 12.37 (s, 1H), 8.37 (s, 1H), 7.72-7.69 (m, 1H), 7.52 (d, J = 8.4 Hz, 1H), 6.86-6.79 (m, 2H), 6.70 (d, J = 8.0 Hz, 1H), 3.73 (s, 3H), 3.70 (s, 3H), 2.58-2.48 (m, 4H), 1.96-1.86 (m, 2H).
MS (ESI) Found; 483[M+H]⁺

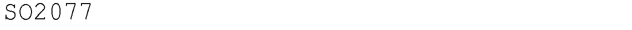

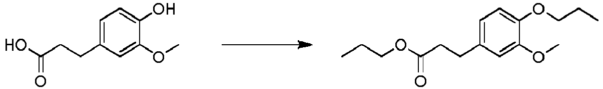

N,N-dimethylformamide solution (5ml) containing 4-hydroxy-3-methoxypheny propionic acid (500 mg, 2.54 mmol) was added with potassium carbonate (881 mg, 6.37 mmol) and 1-bromopropane (692 µl, 7.65 mmol) and stirred over night at room temperature. After completion of the reaction, the solution was diluted with ethyl acetate and washed with water and saturated sodium chloride solution. The solution was dried over anhydrous sodium sulfate and the solvent was removed under reduced pressure. The residue was purified by silica gel column chromatography (n-hexane/ethyl acetate=4/1) and 590 mg of propyl 3-(3-methoxy-4-propoxyphenyl)propanoate was obtained in a yield of 82%.

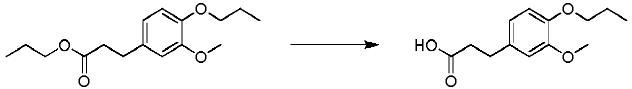

Propyl 3-(3-methoxy-4-propoxyphenyl)propanoate (590 mg, 2.10 mmol) was dissolved in 1,4-dioxane and added with 5 mol/l sodium hydroxide aqueous solution (1.68 ml) and the resulting solution was stirred over night at room temperature. After completion of the reaction, the solution was added with 6 mol/l hydrochloric acid and extracted with ethyl acetate. The organic layer was washed with saturated sodium chloride solution and dried over anhydrous sodium sulfate. The solvent was removed under reduced pressure and 438 mg of 3-(3-methoxy-4-propoxyphenyl)propionic acid was obtained in a yield of 87%.

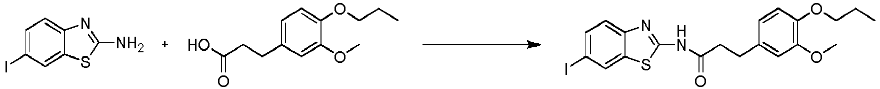

N,N-dimethylformamide solution (3ml) containing 2-amino-6-iodobenzothiazole (200 mg, 0.723 mmol) and 3-(3-methoxy-4-propoxyphenyl)propionic acid (200 mg, 0.839 mmol) was added with N,N-diisopropylethylamine (140 µl, 0.803 mmol) and O-(6-chlorobenzotriazol-1-yl)-N,N,N',N'-tetramethyluronium hexafluorophosphate (360 mg, 0.870 mmol) and stirred over night at room temperature. After completion of the reaction, the solution was diluted with ethyl acetate and washed with saturated sodium bicarbonate solution and saturated sodium chloride solution. The solution was dried over anhydrous sodium sulfate and the solvent was removed under reduced pressure. The residue was recrystallized with ethanol and 217 mg of 2-(3-(3-methoxy-4-propoxyphenyl)propanamide)-6-iodobenzothiazole was obtained in a yield of 60%.
¹H NMR (DMSO-d₆) : δ 12.42 (s, 1H), 8.38-8.37 (m, 1H), 7.72-7.69 (m, 1H), 7.54-7.51 (m, 1H), 6.85-6.82 (m, 2H), 6.72 (d, J = 8.0 Hz, 1H), 3.86-3.82 (m, 2H), 3.72 (s, 3H), 2.87-2.78 (m, 4H), 1.72-1.65 (m, 2H), 094 (t, J = 7.3 Hz, 3H).
MS (ESI) Found; 497[M+H]⁺

## Claims

1. A compound having Formula (II) 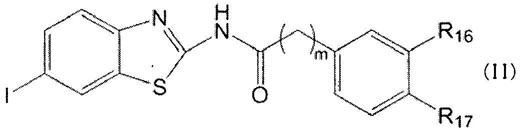 wherein
m is 1 to 4, and
R₁₆ and R₁₇ are independently selected from methoxy, ethoxy and propoxy,
or a salt thereof.

2. The compound of claim 1 or a salt thereof, wherein R₁₆ is methoxy, preferably R₁₇ is methoxy or propoxy.

3. The compound of claim 1 or 2 or a salt thereof, wherein m is 1 to 3.

4. The compound of claim 1 having any one of the following formulas: 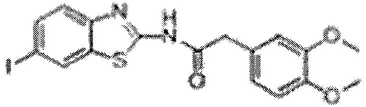 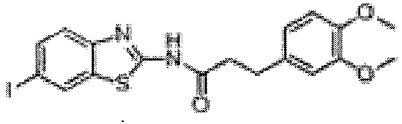 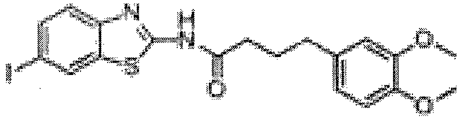 and 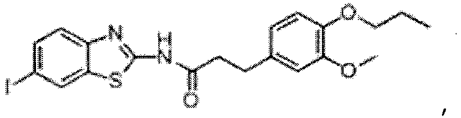 or a salt thereof.

5. A composition for use in promoting cardiac differentiation of a pluripotent stem cell comprising the compound of any one of claims 1-4 or a salt thereof.

6. The composition for use in promoting cardiac differentiation of a pluripotent stem cell of claim 5, wherein the composition comprises the compound 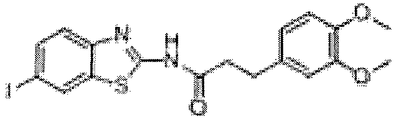 or

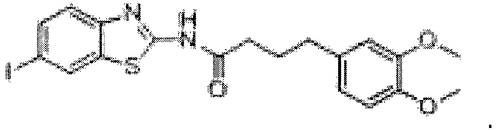

7. The composition for use in promoting cardiac differentiation of a pluripotent stem cell of claim 5 or 6, wherein the composition is added to cardiac differentiation medium to provide a final concentration of the compound of 0.4 to 2 µM.

8. A kit for use in promoting cardiac differentiation of a pluripotent stem cell, said kit comprising the compound of any one of claims 1-4 or a salt thereof.

9. A composition for ex-vivo promoting cardiac differentiation of a pluripotent stem cell comprising the compound of any one of claims 1-4 or a salt thereof.

10. The composition for ex-vivo promoting cardiac differentiation of a pluripotent stem cell of claim 9, wherein the composition comprises the compound 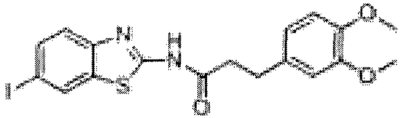 or

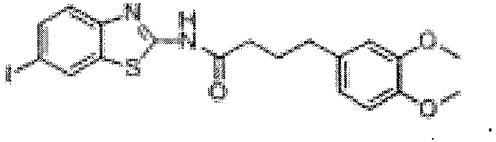

11. The composition for ex-vivo promoting cardiac differentiation of a pluripotent stem cell of claim 9 or 10, wherein the composition is added to cardiac differentiation medium to provide a final concentration of the compound of 0.4 to 2 µM.

12. A kit for ex-vivo promoting cardiac differentiation of a pluripotent stem cell, said kit comprising the compound of any one of claims 1-4 or a salt thereof.

13. A method for inducing cardiac differentiation of a pluripotent stem cell, or for preparing a cardiomyocyte from a pluripotent stem cell, said method comprising culturing the pluripotent stem cell in a medium comprising the compound of any one of claims 1-4 or a salt thereof.

14. The method of claim 13, wherein the method comprises culturing the pluripotent stem cell in a medium comprising the compound 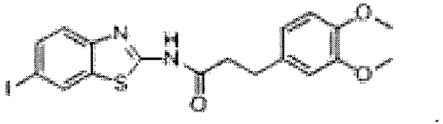 or

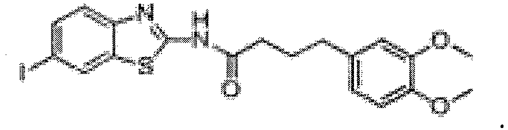

15. The method of claim 13 or 14, wherein the medium comprises the compound at 0.4 to 2 µM.

## Patentansprüche

1. Verbindung mit der Formel (II) 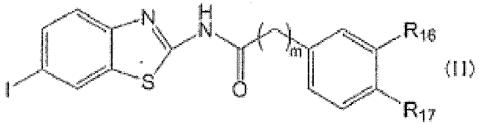 wobei
m 1 bis 4 ist und
R₁₆ und R₁₇ unabhängig aus Methoxy, Ethoxy und Propoxy ausgewählt sind, oder ein Salz davon.

2. Verbindung gemäß Anspruch 1 oder ein Salz davon, wobei R₁₆ Methoxy ist, vorzugsweise R₁₇ Methoxy oder Propoxy ist.

3. Verbindung gemäß Anspruch 1 oder 2 oder ein Salz davon, wobei m 1 bis 3 ist.

4. Verbindung gemäß Anspruch 1, die eine der folgenden Formeln aufweist: 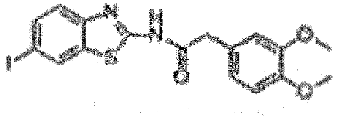 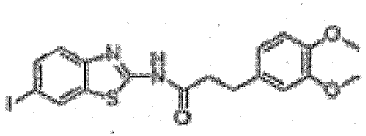 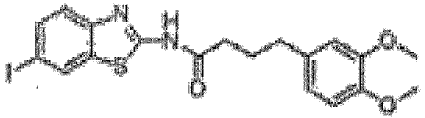 und 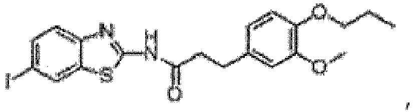 oder ein Salz davon.

5. Zusammensetzung zur Verwendung bei der Förderung der kardialen Differenzierung einer pluripotenten Stammzelle, die die Verbindung gemäß einem der Ansprüche 1 bis 4 oder ein Salz davon umfasst.

6. Zusammensetzung zur Verwendung bei der Förderung der kardialen Differenzierung einer pluripotenten Stammzelle gemäß Anspruch 5, wobei die Zusammensetzung die Verbindung 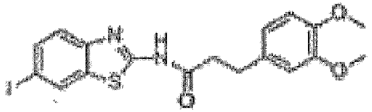 oder 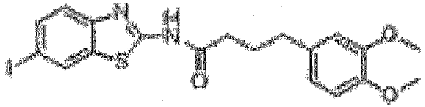 umfasst.

7. Zusammensetzung zur Verwendung bei der Förderung der kardialen Differenzierung einer pluripotenten Stammzelle gemäß Anspruch 5 oder 6, wobei die Zusammensetzung so zu einem kardialen Differenzierungsmedium gegeben wird, dass man eine Endkonzentration der Verbindung von 0,4 bis 2 µM erhält.

8. Kit zur Verwendung bei der Förderung der kardialen Differenzierung einer pluripotenten Stammzelle, wobei der Kit die Verbindung gemäß einem der Ansprüche 1 bis 4 oder ein Salz davon umfasst.

9. Zusammensetzung zur ex-vivo-Förderung der kardialen Differenzierung einer pluripotenten Stammzelle, die die Verbindung gemäß einem der Ansprüche 1 bis 4 oder ein Salz davon umfasst.

10. Zusammensetzung zur ex-vivo-Förderung der kardialen Differenzierung einer pluripotenten Stammzelle gemäß Anspruch 9, wobei die Zusammensetzung die Verbindung 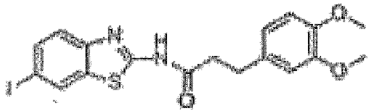 oder 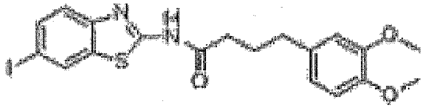 umfasst.

11. Zusammensetzung zur ex-vivo-Förderung der kardialen Differenzierung einer pluripotenten Stammzelle gemäß Anspruch 9 oder 10, wobei die Zusammensetzung so zu einem kardialen Differenzierungsmedium gegeben wird, dass man eine Endkonzentration der Verbindung von 0,4 bis 2 µM erhält.

12. Kit zur ex-vivo-Förderung der kardialen Differenzierung einer pluripotenten Stammzelle, wobei der Kit die Verbindung gemäß einem der Ansprüche 1 bis 4 oder ein Salz davon umfasst.

13. Verfahren zum Induzieren der kardialen Differenzierung einer pluripotenten Stammzelle oder zur Herstellung eines Kardiomyocyten aus einer pluripotenten Stammzelle, wobei das Verfahren das Kultivieren der pluripotenten Stammzelle in einem Medium umfasst, das die Verbindung gemäß einem der Ansprüche 1 bis 4 oder ein Salz davon umfasst.

14. Verfahren gemäß Anspruch 13, wobei das Verfahren das Kultivieren der pluripotenten Stammzelle in einem Medium umfasst, das die Verbindung 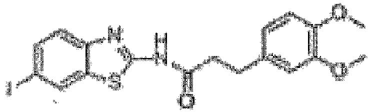 oder 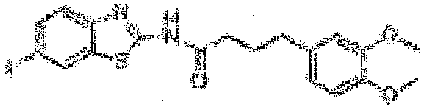 umfasst.

15. Verfahren gemäß Anspruch 13 oder 14, wobei das Medium die Verbindung in einer Menge von 0,4 bis 2 µM umfasst.

## Revendications

1. Composé ayant la formule (II) 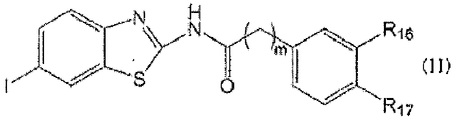 où
m est égal à de 1 à 4, et
R₁₆ et R₁₇ sont indépendamment choisis parmi un groupe méthoxy, éthoxy et propoxy, ou sel de celui-ci.

2. Composé selon la revendication 1 ou sel de celui-ci, où R₁₆ est le groupe méthoxy, de préférence R₁₇ est le groupe méthoxy ou propoxy.

3. Composé selon la revendication 1 ou 2 ou sel de celui-ci, où m est égal à de 1 à 3.

4. Composé selon la revendication 1 ayant une des formules suivantes : 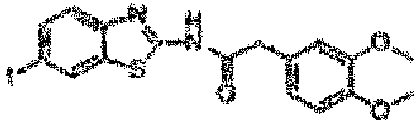 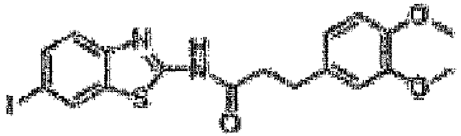 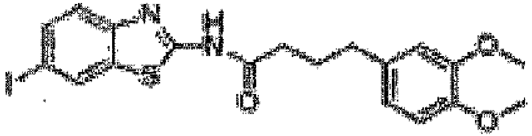 et 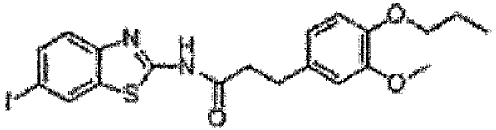 ou sel de celui-ci.

5. Composition pour une utilisation dans la promotion de différenciation cardiaque d'une cellule souche pluripotente comprenant le composé selon l'une quelconque des revendications 1-4 ou un sel de celui-ci.

6. Composition pour une utilisation dans la promotion de différenciation cardiaque d'une cellule souche pluripotente selon la revendication 5, où la composition comprend le composé 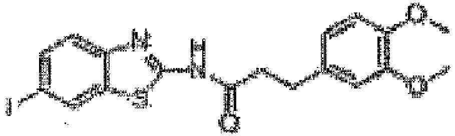 ou

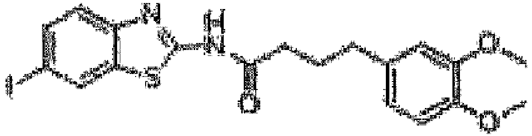

7. Composition pour une utilisation dans la promotion de différenciation cardiaque d'une cellule souche pluripotente selon la revendication 5 ou 6, où la composition est ajoutée à un milieu de différenciation cardiaque pour fournir une concentration finale du composé de 0,4 à 2 µM.

8. Kit pour une utilisation dans la promotion de différenciation cardiaque d'une cellule souche pluripotente, ledit kit comprenant le composé selon l'une quelconque des revendications 1-4 ou un sel de celui-ci.

9. Composition pour la promotion ex-vivo de différenciation cardiaque d'une cellule souche pluripotente comprenant le composé selon l'une quelconque des revendications 1-4 ou un sel de celui-ci.

10. Composition pour la promotion ex-vivo de différenciation cardiaque d'une cellule souche pluripotente selon la revendication 9, où la composition comprend le composé 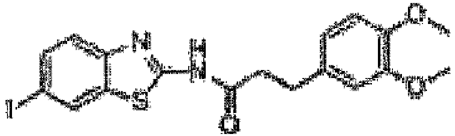 ou

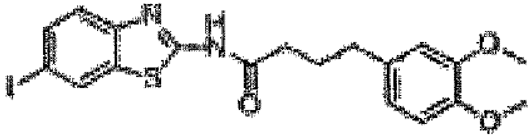

11. Composition pour la promotion ex-vivo de différenciation cardiaque d'une cellule souche pluripotente selon la revendication 9 ou 10, où la composition est ajoutée à un milieu de différenciation cardiaque pour fournir une concentration finale du composé de 0,4 à 2 µM.

12. Kit pour la promotion ex-vivo de différenciation cardiaque d'une cellule souche pluripotente, ledit kit comprenant le composé selon l'une quelconque des revendications 1-4 ou un sel de celui-ci.

13. Procédé pour l'induction de différenciation cardiaque d'une cellule souche pluripotente, ou pour la préparation d'un cardiomyocyte à partir d'une cellule souche pluripotente, ledit procédé comprenant la mise en culture de la cellule souche pluripotente dans un milieu comprenant le composé selon l'une quelconque des revendications 1-4 ou un sel de celui-ci.

14. Procédé selon la revendication 13, où le procédé comprend la mise en culture de la cellule souche pluripotente dans un milieu comprenant le composé 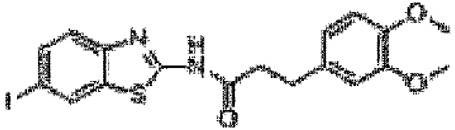 ou

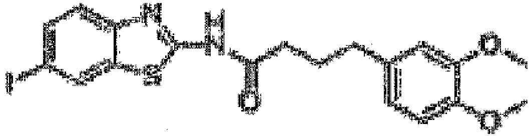

15. Procédé selon la revendication 13 ou 14, où le milieu comprend le composé à de 0,4 à 2 µM.
